# EUROPEAN PATENT SPECIFICATION

(11) **EP 3 887 819 B1**
(45) Date of publication and mention of the grant of the patent: **01.06.2022**
(21) Application number: 19813273.0
(22) Date of filing: 27.11.2019
(51) Int. Cl.: G01N 30/02, G01N 30/46, G01N 30/78, G01N 33/00, G01N 33/497, A61B 5/00, A61B 5/097, G01N 1/40, A61B 5/08, G01N 1/22, G01N 30/06

(54) **GAS ANALYSIS SAMPLING WITH OVERLOAD DETECTION**
GAS PROBENAHME FÜR DIE ANALYSE MIT FESTSTELLUNG DER ÜBERLADUNG
ÉCHANTILLONNAGE D'ANALYSE DE GAZ AVEC DÉTECTION DE SURCHARGE

(30) Priority: 28.11.2018 US 201862772128 P
(43) Date of publication of application: 06.10.2021
(73) Proprietor: Koninklijke Philips N.V., 5656 AG Eindhoven (NL)
(72) Inventor: WEDA, Johannes, 5656 AE Eindhoven (NL); NIJSEN, Tamara, Mathea, Elisabeth, 5656 AE Eindhoven (NL); KNOBEL, Hugo, Hubertus, 5656 AE Eindhoven (NL); KLOOTWIJK, Johan, Hendrik, 5656 AE Eindhoven (NL); VERSCHUEREN, Alwin, Rogier, Martijn, 5656 AE Eindhoven (NL)
(74) Representative: Philips Intellectual Property & Standards
(86) International application number: PCT/EP2019/082806
(87) International publication number: WO 2020/109424

(56) References cited:
- WO-A1-2017/216079
- US-A1- 2003 109 794
- US-A1- 2015 335 267
- US-A1- 2017 303 822

## Description

### FIELD OF THE INVENTION

The invention relates generally to sampling and analysis of gas, e.g. medical sampling and analysis of gas, e.g. breath exhaled from a person or gas based on samples from skin, urine, feces or other body fluids. Specifically, the invention relates to a method and a device suited for sampling of gas in a sorbent tube without overloading the sorbent tube.

### BACKGROUND OF THE INVENTION

Exhaled breath analysis in health and disease is an area of growing clinical interest. Using breath as a biological sample is appealing, because breath-collection is cheap, easy to perform and non-invasive. Volatile Organic Compounds (VOCs) are excreted from the skin, urine, feces and most notably via exhaled breath. Besides pulmonary origin, VOCs also originate from the blood, reflecting any physiological, pathological or pathogen related biochemical processes throughout the body. As such exhaled breath analysis may allow metabolic fingerprinting of disease processes anywhere inside the body.

Typically, in a clinical setting, a fixed predetermined breath volume is flown through a sorbent tube to avoid the problem of overloading the sorbent tube, and thus destroying the sample. Even with this practice, overloading of the sorbent tubes is still a problem, rendering a high number of breath samples useless for further analysis, since the overloading disturbs the quantitative capturing of VOCs. Failed measurements need to be redone, i.e. sampling as well as analysis (e.g. with gas chromatography-mass spectrometry systems), thus leading to time delays, extra burden and costs, as well as an increased risk. Sometimes it may not even be impossible to redo a measurement, and thus leading to a fatal loss of the sample.

### SUMMARY OF THE INVENTION

Following the above, the inventors of the present invention have appreciated that an improved device and method for gas sampling in sobent tubes is advantageous. Specifically, such method and device preferably solves the above mentioned problem of overloaded sorbent tubes during the sampling process.

In a first aspect, the invention provides a gas sampling device arranged to load an associated sorbent tube with volatile organic compound (VOC) from a gas sample, e.g. breath of a human, in a sampling process, the gas sampling device comprising
- a gas inlet arranged to receive the gas sample,
- a port arranged to receive the associated sorbent tube,
- a gas circuit connected to the gas inlet and being arranged to provide a flow path to guide the gas sample to the port, so as to allow the gas sample to load the associated sorbent tube,
- at least one VOC detector arranged to sense gas in the flow path upstream or downstream of the port, the VOC detector being arranged to generate an output signal in accordance with a sensed VOC compound concentration, and
- a control module connected to the at least one VOC detector, and being arranged to calculate a measure indicative of a cumultative sum of VOCs in response to the output signal from the detector, and to compare said measure with a predetermined threshold value, and wherein the control module is arranged to generate an output in case said measure exceeds the predetermined threshold value.

Such gas sampling device is advantageous, since the inventors have realized that the use of a rather simple VOC detector, it is possible to monitor the amount of VOC being applied to the sorbent tube during a sampling process. When a predetermined cumultative sum of VOCs has been reached for the sampling process, determined by the threshold value, the control module generates an output to indicate that the sampling process should stop. Hereby, the problem of overloading of the sorbent tube can be avoded. The output from the control module could be used to generate a signal to a user to stop the sampling process in a manual way. Alternatively or additionally, the output from the control module may control a valve in the flow path, and/or control a pump serving to force the gas sample through the sorbent tube. Thus, in a easy way, it is possible to provide an automatic stop of the sampling process, e.g. to close a vale and/or to stop a pump, thereby avoiding overloading of the sorbent tube.

The threshold value may be adjustable or selectable, so as to allow the device to be suited for different types of sorbent tubes with different VOC capacity. The threshold value may be manually entered by the user, or it may be automatically entered by the device by means of a code reader for reading a code a type code on the sorbent tube, e.g. bar code, a QR code or the like, and selecting the threshold accordingly, e.g. based on a stored list of values.

Since the necessary VOC detector is a low cost element, the invention is suitable for low cost gas sampling devices, e.g. handheld breath sampling devices.

The invention is based on the insight that instead of controlling the gas sampling process based on a predetermined gas volume, it is possible to detect the total VOC applied to the sorbent tube, and thus stop the gas sampling process when the VOC capacity of the sorbent tube has been reached.

In the following, preferred embodiments or features of the first aspect will be described.

The gas sampling device may comprise a VOC detector arranged in the flow path upstream and/or downstream of the port. Especially, it may be preferred to have a first VOC detector upstream and a second VOC detector downstream of the port. An upstream detector is most preferably non-destroying, preferably in the form of a Thermal Conductivity Detector, as known by the skilled person. A downstream detector need not be non-destroying, and preferably it is a non-selective VOC detector. Examples for the downstream detector are: chemiresistor (e.g. metal-oxide, MOX) detectors, photo-ionization detectors (PID), quartz crystal microbalance (QCM) detectors or micro-cantilever detectors, as known by the skilled person.

In preferred embodiment, the gas sampling device comprises a controllable pump, e.g. driven by an electric motor, and being arranged in the flow path to generate a flow to force the gas sample from the gas inlet to the port. Preferably, the pump is arranged downstream of the port to avoid particles from the pump to enter the sorbent tube. Preferably, the controllable pump is arranged to stop to generate said flow in response to the output from the control module, so as to stop loading of the associated sorbent tube. Thereby, an automatic stop of the gas sampling process is provided to avoid overloading of the sorbent tube.

In another embodiment, the gas sampling device comprises a controllable value in the flow path to stop a flow of gas to the port in response to the output from the control module, so as to stop loading of the associated sorbent tube. This is an alternative way of providing an automatic stop of the gas sampling process.

The control module may be arranged to generate an output to indicate to a user that the associated sorbent tube is loaded, in case said measure exceeds the predetermined threshold value. E.g. such output may be in the form of a visual and/or auditive output, e.g. using a display, an LED indicator and/or a loudspeaker. The user can then manually stop the gas sampling process.

The control module may be arranged to select between a plurality of different threshold values, thereby allowing the device to adapt to different types of sorbent tubes with different VOC capacities. Especially, the gas sampling device may comprise a code reader arranged to read a code of the associated sorbent tube, and wherein the control module is arranged to receive an output from the code reader indicative of the code, and to select said threshold value accordingly. This can allow an automatic adaption to the VOC loading capacity of a given sorbent tube mounted in the port of the device. Alternatively, or additionally, a user input device, e.g. a touch screen or a knob or buttons, can be used to receive an input from the user indicative of a type of the assotiaced sorbent tube, and wherein the control module is arranged to select said threshold vaue according to said input from the user. This allows the user to manualy input the actual sorbent tube type, and the gas sampling device can then adapt its gas sampling stop criterion accordingly, e.g. based on stored threshold values for different types of sorbent tubes.

The gas sampling device may comprising a flow meter arranged in the flow path to measure a volume of gas passing the port, and to generate an output accordingly. The control module is then arranged to receive the output from the flow meter, and to calculate a value indicative of a total volume of gas having passed the port during a sampling process, and to store said value in a database. In existing methods, this volume is typically the same, but the gas sampling process stop criterion according to the invention is determined based on the VOC amount, and not the gas volume, so the actual gas volume in a given gas sampling process may be interesting for later analysis, especially in order to be able to calculate correct concentrations.

It is to be understood that the gas circuit to provide the flow path preferably pipes etc. with properties such as known by the skilled person.

The control module preferably comprises a processor programmed to perform a gas sampling process algorithm comprising monitoring the output from the one or more VOC detectors during the gas sampling process, and to continuously, or at least regularly, calculate an updated value of cumultative sum of VOC, so as to monitor when the threshold value is exceeded.

In a second aspect, the invention provides a handheld breath sampling device comprising a gas sampling device according to the first aspect, wherein the gas input is arranged to receive exhaled breath. The gas inlet may be in the form of a mouth piece arranged on an exterior part of a casing of the handheld device, so as to allow the subject, a person or an animal, to directly breathe into the mouthpiece and thus provide a gas sample to be sampled. Other tube fittings may be used for connection to receive breathed air from a mechanical ventilator (in intensive care units or OR) to which the subject is connected. Still further, the gas inlet of the device may be arranged for mounting of a gas bag with the gas sample to be analyzed.

In a third aspect, the invention a method for sampling gas in a sorbent tube in a sampling process, the method comprising
- receiving a gas sample at a gas inlet,
- guiding the gas sample from the gas inlet to load the sorbent tube,
- detecting a volatile organic compound concentration with a detector during the sampling process,
- monitoring a measure indicative of a cumultative sum of volatile organic compounds in response to the detected volatile organic compound concentration during the sampling process,
- comparing said measure with a predetermined threshold value, and
- stopping the sampling process in case said measure exceeds the predetermined threshold value.

Especially, the steps of monitoring, comparing, and stopping may be performed automatically by a control module comprising a processor programmed accordingly.

In general, it is appreciated that the various aspects of the invention may be combined and coupled in any way possible within the scope of the invention. These and other aspects, features and/or advantages of the invention will be apparent from and elucidated with reference to the embodiments described hereinafter.

### BRIEF DESCRIPTION OF THE DRAWINGS

Embodiments of the invention will be described, by way of example only, with reference to the drawings, in which
FIG. 1 illustrates graphs indicating the problem with overloading of sorbent tubes,
FIG. 2 illustrates in schematic form a gas sampling device embodiment,
FIG. 3 illustrates a breath sampling device embodiment, and
FIG. 4 illustrates steps of a method embodiment.

### DESCRIPTION OF EMBODIMENTS

Sorbent tubes are typically filled by letting a predetermined gas volume flow through the sorbent tube. If the volume is too low, a low amount of VOCs is captured, reducing the sensitivity of the measurement. On the other hand, if the volume is too high, the sorbent tube is overloaded, abundant VOCs can significantly alter the caption of other VOCs. The disturbance of the subsequent chromatogram analysis can be so severe that the concentration of the VOCs cannot be quantitatively determined, thus leading to a failed measurement that needs to be redone.

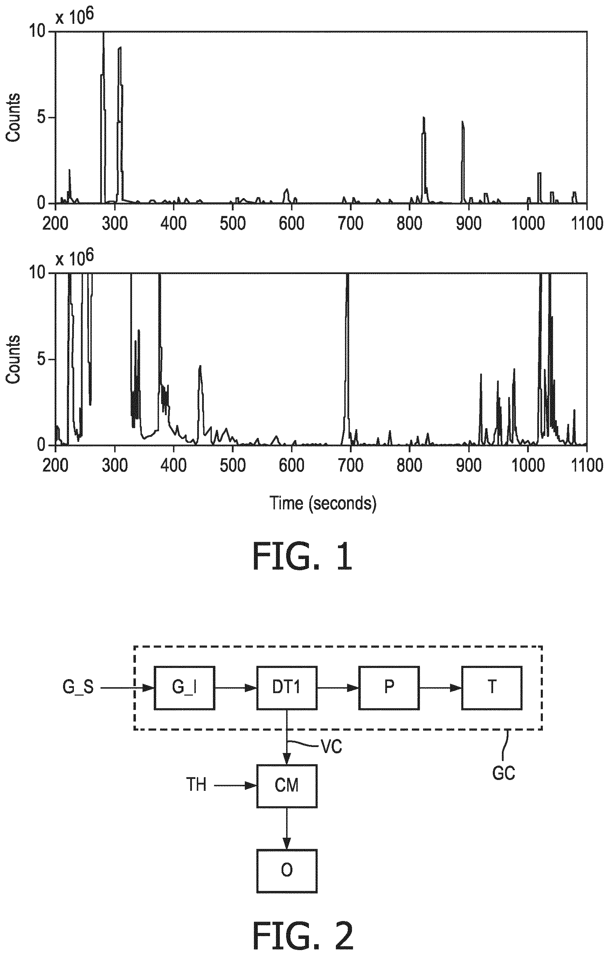

FIG. 1 illustrates two graphs showing examples from a clinical trial with gas sampling for loading of sorbent tubes. The two graphs show count versus time, i.e. chromatograms. The top graph indicates a well captured breath sample from an mechanically ventilated ICU patient. The bottom chromatogram, from another mechanically ventilated ICU patient, is severely distorted by the abundant presence of ethanol. The amount of other VOCs, certainly at the low retention times, cannot be quantitatively determined, rendering this sample useless for further analysis. This problem can be solved by the present invention, where a VOC detector is used to provide a gas sampling process stop criterion.

FIG 2. illustrates a block diagram of elements of a gas sampling device embodiment arranged to load a sorbent tube T with VOC from a gas sample G_S in a sampling process. The device has a gas inlet G_I to receive the gas sample G_S for entering a gas circuit GC. A port P is arranged to receive the sorbent tube T, e.g. by manual entering of the tube T by a user. The gas circuit GC connects the gas inlet G_I and provides a flow path to guide the gas sample G_S to the port P, so as to allow the gas sample G_S to load the associated sorbent tube T. A VOC detector DT1, here shown upstream of the port P and thus upstream of the tube T, is arranged to sense gas in the flow path, and the VOC detector DT1 generates an output signal VC in accordance with a sensed VOC concentration.

A control module CM comprising a processor, is connected to the VOC detector DT1 and receives the output signal VC from the detector DT1 and calculates in response a measure indicative of a cumultative sum of VOCs. Thus, the sum of VOCs having passed the detector DT1 during the sampling process is determined and preferably continuously monitored and compared with a predetermined threshold value TH which has been set for the actual sorbent tube T. The control module CM then generates an output O in case the measured cumultative sum of VOCs measure exceeds the threshold value TH. Thus, by proper selection of the threshold value TH, it can be ensured that the control module CM generates an output O, e.g. an electric output to other elements such as a valve or a pump or a light or sound generator, so as to ensure that the gas sampling process is stopped at the time where the sorbent tube T has been loaded, but before it is overloaded. This can either be done fully automatic or by means of signaling to the user who can then manually stop the gas sampling process.

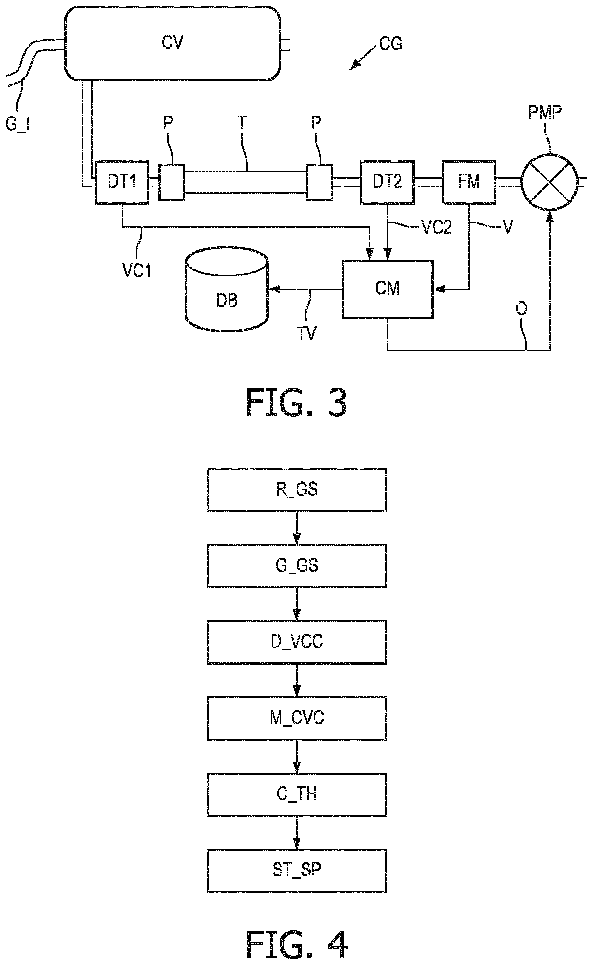

FIG. 3 illustrates a schematic overview of a breath sampling device embodiment, where a gas circuit GC provides a flow path to connect a gas inlet G_I with a sorbent tube T mounted in the port P. A person exhales in the gas inlet pipe G_I, e.g. a mouth piece, and the breath then enters a cavity CV. The cavity CV can be a hose connecting an ICU patient to a mechanical ventilator in a hospital settings, and the breath is then sampled using a small side stream. Otherwise, the cavity CV be a sampling bag such as a Tedlar bar, where the person exhales into the bag, and the breath is subsequently sampled onto the sorbent tube T from this bag (after the person has finished filling the bag with exhaled breath). Further, the cavity CV can be a mouth-nose cover into which the person exhales. Still further, the cavity CV can be a long tube where the sorbent tube is filled by sampling from this tube.

From the cavity CV, the sorbent tube T is loaded by a controllable pump PMP which generates a flow of gas through the sorbent tube T. The pump PMP is controlled, i.e. at least stopped, e.g. also started, by a control module CM, and the pump PMP can provide a fixed flow rate through the sorbent tube T. The pump can be controlled by a mass flow controller that tunes the flow rate of the gas to a predetermined value. The pump can best be positioned downstream of the sorbent tube T such that VOCs originating from the patient cannot be trapped in the pump materials, and VOCs emerging from the pump PMP cannot be adsorbed in the sorbent tube T.

An upstream VOC detector DT1 and a downstream VOC detector DT2 are connected to the control module CM which receive their respective output VC1, VC2 indicative of momentary VOC concentrations. The control module then calculates a cumultative sum of VOCs accordingly, and compares this with a predetermined threshold value. When the threshold value is exceeded or at least reached, the control module generates an output O to cause the pump PMP to stop, and thus the sampling process stops.

The upstream VOC detector DT1 is most preferably a Thermal Conductivity Detector, and the downstream VOC detector DT2 may be such as: a chemiresistor (e.g. metal-oxide, MOX) detector, a photo-ionization detector (PID), a quartz crystal microbalance (QCM) detector, or a micro-cantilever detector.

A flow meter FM is arranged to continuously measure gas flow and generate an output V accordingly, and the control module CM receives this output V and calculates in response the total volume TV of gas during the gas sampling process. This value TV is then stored in a database DB to allow later use during the VOC composition analysis of the loaded sorbent tube T.

FIG. 4 illustrates steps of an embodiment of a method for sampling gas in a sorbent tube in a sampling process. The method comprises receiving R_GS a gas sample, e.g. exhaled breath, at a gas inlet. The gas sample is then guided G_GS from the gas inlet to load the sorbent tube via tubes or pipes, and the sorbent tude is preferably connected to the tubes or pipes via a port to allow removing the sorbent tube for further analysis of VOC composition. During the sampling process, VOC concentrations is detected D_VCC with a detector either upstream or downstream of the sorbent tube. During the sampling process, a measure indicative of a cumultative sum of VOCs is monitored M_CVC in response to the detected VOC concentration. E.g. this may be performed by adding or integrating VOC concentration values received from the VOC detector, wherein the cumulative sum of VOCs has been reset at the start of the sampling process. Continuously, or at least with a small time interval, a process of comparing C_TH said measure indicative of a cumultative sum of VOCs with a predetermined threshold value is repeated. The sampling process is stopped ST_SP, in case it is observed that said measure exceeds the predetermined threshold value, or an equivalent rule for stopping has been set up based on a relation between said measure and a threshold value. The method may include the initial step of selecting the threshold value in response to information about the sorbent tube, especially its VOC capacity, so as to ensure that the sampling process is continued until the sorbent tube is fully loaded, but not overloaded. A user, e.g. medial personnel, may manually enter the type or number or VOC capacity of the sorbent tube, or the sorbent tube type may be automatically read by a code reader, or the like, and the threshold value can then automatically be selected accordingly.

To sum up, the invention provides a gas sampling device suited for e.g. sampling of exhaled breath from a subject in a sorbent tube with VOC from a gas sample received at a gas inlet. A gas circuit provides a flow path from the gas inlet to a port where the sorbent tube is connected, so as to allow the gas sample to load the sorbent tube. A VOC detector in the flow path serves to detect VOC concentration during the sampling process. A control module continuously calculates a measure of cumultative sum of VOCs based on the output from the VOC detector, and this measure is then compared with a predetermined threshold value. When the measure exceeds the threshold value, the control module generates an output, and the sampling process is then preferably stopped accordingly, e.g. by controlling a pump or value in the flow path to stop gas flow in response to the output. Such device can eliminate the problem with overloading of sorbent tubes, which leads to invalid gas measurements that needs to be redone. Thus, the device can save time, costs, and risks.

While the invention has been illustrated and described in detail in the drawings and foregoing description, such illustration and description are to be considered illustrative or exemplary and not restrictive; the invention is not limited to the disclosed embodiments.

## Claims

1. A gas sampling device arranged to load an associated sorbent tube (T) with volatile organic compound from a gas sample (G_S) in a sampling process, the gas sampling device comprising
- a gas inlet (G_I) arranged to receive the gas sample (G_S),
- a port (P) arranged to receive the associated sorbent tube (T),
- a gas circuit (GC) connected to the gas inlet (G_I) and being arranged to provide a flow path to guide the gas sample (G_S) to the port (P), so as to allow the gas sample (G_S) to load the associated sorbent tube (T),
- at least one volatile organic compound detector (DT1) arranged to sense gas in the flow path upstream or downstream of the port (P), the detector (DT1) being arranged to generate an output signal (VC) in accordance with a sensed volatile organic compound concentration, and
- a control module (CM) connected to the at least one volatile organic compound detector (DT1), and being arranged to calculate a measure indicative of a cumultative sum of volatile organic compounds in response to the output signal (VC) from the detector (DT1), and to compare said measure with a predetermined threshold value (TH), and wherein the control module (CM) is arranged to generate an output (O) in case said measure exceeds the predetermined threshold value (TH).

2. The gas sampling device according to claim 1, comprising a volatile organic compound detector (DT1) arranged in the flow path upstream of the port (P).

3. The gas sampling device according to claim 1, comprising a volatile organic compound detector (DT2) arranged in the flow path downstream of the port (P).

4. The gas sampling device according to claim 1, comprising a first volatile organic compound detector (DT1) arranged in the flow path upstream of the port (P), and a second volatile organic compound detector (DT2) arranged in the flow path downstream of the port (P).

5. The gas sampling device according to claim 1, comprising a controllable pump (PMP) arranged in the flow path and being arranged to generate a flow to force the gas sample (G_S) from the gas inlet (G_I) to the port (P).

6. The gas sampling device according to claim 5, wherein the controllable pump (PMP) is arranged to stop to generate said flow in response to the output (O) from the control module (CM), so as to stop loading of the associated sorbent tube (T).

7. The gas sampling device according to claim 1, comprising a controllable value in the flow path to stop a flow of gas to the port (P) in response to the output (O) from the control module (CM), so as to stop loading of the associated sorbent tube (T).

8. The gas sampling device according to claim 1, wherein the control module (CM) is arranged to generate an output (O) to indicate to a user that the associated sorbent tube (T) is loaded, in case said measure exceeds the predetermined threshold value (TH).

9. The gas sampling device according to claim 1, wherein the control module (CM) is arranged to select between a plurality of different threshold values (TH).

10. The gas sampling device according to claim 9, comprising a code reader arranged to read a code of the associated sorbent tube (T), and wherein the control module (CM) is arranged to receive an output from the code reader indicative of the code, and to select said threshold value (TH) accordingly.

11. The gas sampling device according to any of claim 9, comprising a user input device arranged to receive an input from a user indicative of a type of the assotiaced sorbent tube (T), and wherein the control module (CM) is arranged to select said threshold vaue (TH) according to said input from the user.

12. The gas sampling device according to claim 1, comprising a flow meter (FM) arranged in the flow path to measure a volume of gas passing the port (P), and to generate an output (V) accordingly.

13. The sampling device according to claim 12, wherein the control module (CM) is arranged to receive the output (V) from the flow meter (FM), and to calculate a value indicative of a total volume (TV) of gas having passed the port (P) during a sampling process, and to store said value in a database (DB).

14. A handheld breath sampling device comprising a gas sampling device according to claim 1, wherein the gas input (G_I) is arranged to receive exhaled breath (G_S).

15. A method for sampling gas in a sorbent tube in a sampling process, the method comprising
- providing a gas sampling device as defined in claims 1-13,
- receiving (R_GS) a gas sample at the gas inlet,
- guiding (G_GS) the gas sample from the gas inlet to load the sorbent tube,
- detecting (D VCC) a volatile organic compound concentration with the detector during the sampling process,
- monitoring (M_CVC) a measure indicative of a cumultative sum of volatile organic compounds in response to the detected volatile organic compound concentration during the sampling process,
- comparing (C_TH) said measure with a predetermined threshold value, and
- stopping (ST_SP) the sampling process in case said measure exceeds the predetermined threshold value.

## Patentansprüche

1. Ein Gerät zur Gasanalyse, angeordnet, um ein damit zusammenhängendes Sorptionsrohr (T) mit einer flüchtigen organischen Verbindung aus einer Gasprobe (G_S) in einem Vorgang der Probenahme zu beladen. Das Gerät zur Gasanalyse umfasst
- einen Gaseinlass (G_I), angeordnet, um die Gasprobe (G_S) aufzunehmen,
- eine Öffnung (P), angeordnet, um das damit zusammenhängende Sorptionsrohr (T) aufzunehmen,
- einen Gaskreislauf (GC), an den Gaseinlass (G_I) angeschlossen und angeordnet, um einen Pfad für den Durchfluss zu bieten, um die Gasprobe (G_S) zur Öffnung (P) zu leiten, um es der Gasprobe (G_S) zu ermöglichen, das damit zusammenhängende Sorptionsrohr (T) zu beladen,
- wenigstens einen Detektor für flüchtige organische Verbindungen (DT1), angeordnet, um Gas im Pfad des Durchflusses stromauf- oder abwärts der Öffnung (P) zu spüren. Der Detektor (DT1) ist angeordnet, ein Ausgabesignal (VC) in Übereinstimmung mit einer gespürten Konzentration einer flüchtigen organischen Verbindung zu erzeugen,
und
- ein Steuermodul (CM), das mit dem mindestens einen Detektor für flüchtige organische Verbindungen (DT1) verbunden und dafür eingerichtet ist, als Antwort auf das Ausgangssignal (VC) von dem Detektor (DT1) ein Maß zu berechnen, das eine kumulative Summe von flüchtigen organischen Verbindungen anzeigt, und um das besagte Maß mit einem vorbestimmten Schwellenwert (TH) zu vergleichen, und wobei das Steuermodul (CM) angeordnet ist, um eine Ausgabe (O) zu erzeugen, falls das besagte Maß den vorbestimmten Schwellenwert (TH) überschreitet.

2. Das Gerät zur Gasanalyse gemäß Anspruch 1, welches einen Detektor für eine flüchtige organische Verbindung (DT1) umfasst, im Durchflusspfad stromaufwärts der Öffnung (P) angeordnet.

3. Das Gerät zur Gasanalyse gemäß Anspruch 1, welches einen Detektor für eine flüchtige organische Verbindung (DT2) umfasst, im Durchflusspfad stromabwärts der Öffnung (P) angeordnet.

4. Das Gerät zur Gasanalyse gemäß Anspruch 1, welches einen ersten Detektor für eine flüchtige organische Verbindung (DT1) umfasst, im Durchflusspfad stromaufwärts der Öffnung (P) angeordnet, und einen zweiten Detektor für eine flüchtige organische Verbindung (DT2) umfasst, im Durchflusspfad stromabwärts der Öffnung (P) angeordnet.

5. Das Gerät zur Gasanalyse gemäß Anspruch 1, welches eine steuerbare Pumpe (PMP) umfasst, die im Durchflusspfad angeordnet ist und so angeordnet ist, dass sie die Gasprobe (G_S) zwangsweise vom Gaseingang (G_I) zur Öffnung (P) transportiert.

6. Das Gerät zur Gasanalyse gemäß Anspruch 5, wobei die steuerbare Pumpe (PMP) angeordnet ist, den besagten erzeugten Gasdurchfluss in Reaktion auf die Ausgabe (O) aus dem Steuermodul (CM) anzuhalten und somit die Beladung der damit zusammenhängenden Sorptionsrohrs (T) zu stoppen.

7. Das Gerät zur Gasanalyse gemäß Anspruch 1, ein steuerbares Ventil im Durchflusspfad umfassend, um den Gasdurchfluss (P) in Reaktion auf die Ausgabe (O) aus dem Steuermodul (CM) anzuhalten und somit die Beladung der damit zusammenhängenden Sorptionsrohrs (T) zu stoppen.

8. Das Gerät zur Gasanalyse gemäß Anspruch 1, wobei das Steuermodul (CM) angeordnet ist, eine Ausgabe (O) zu erzeugen, um einem Benutzer anzuzeigen, dass das damit zusammenhängende Sorptionsrohr (T) beladen ist, falls das besagte Maß den vorbestimmten Schwellwert (TH) überschreitet.

9. Das Gerät zur Gasanalyse gemäß Anspruch 1, wobei das Steuermodul (CM) angeordnet ist, eine Auswahl zwischen einer Vielzahl von verschiedenen Schwellwerten (TH) zu treffen.

10. Das Gerät zur Gasanalyse gemäß Anspruch 9, welches ein Code-Lesegerät umfasst, um einen Code vom damit zusammenhängenden Sorptionsrohr (T) abzulesen, und wobei das Steuermodul (CM) angeordnet ist, eine Ausgabe vom Code-Lesegerät zu empfangen, welche den Code anzeigt, und entsprechend dazu einen Schwellwert (TH) auszuwählen.

11. Das Gerät zur Gasanalyse gemäß einem beliebigen Anspruch 9, umfassend eine Vorrichtung zur Benutzereingabe, so angeordnet, dass sie eine Eingabe von einem Benutzer empfängt, die einen Typ des zugehörigen Sorptionsrohrs (T) angibt, und wobei das Steuermodul (CM) so angeordnet ist, dass es den besagten Schwellwert (TH) gemäß der besagten Eingabe vom Benutzer auswählt.

12. Das Gerät zur Gasanalyse gemäß Anspruch 1, welches ein Durchfluss-Messgerät (FM) umfasst, im Durchflusspfad angeordnet, um ein Gasvolumen, das durch die Öffnung (P) hindurchströmt, zu messen und eine entsprechende Ausgabe (V) zu erzeugen.

13. Das Analysegerät nach Anspruch 12, wobei das Steuermodul (CM) angeordnet ist, um die Ausgabe (V) von dem Durchflussmesser (FM) zu empfangen und einen Wert zu berechnen, der ein Gesamtvolumen (TV) von Gas angibt, das durch die Öffnung (P) während einer Probenahme hindurchgeströmt ist, und diesen Wert in einer Datenbank (DB) zu speichern.

14. Ein mit der Hand zu haltendes Gerät zur Atemanalyse, welches ein Gasanalysegerät entsprechend Anspruch 1 umfasst, wobei der Gaseintritt (G_I) angeordnet ist, ausgeatmetes Atemgas zu empfangen (G_S).

15. Eine Methode zur Gasanalyse in einem Sorptionsrohr in einem Analyseverfahren, die Methode umfasst:
- Zurverfügungstellung eines Gasanalysegerätes wie in Ansprüchen 1-13 definiert,
- Empfangen (R_GS) einer Gasprobe am Gaseingang,
- Führen (G_GS) einer Gasprobe vom Gaseingang zur Beladung des Sorptionsrohrs,
- Erkennen (D_VCC) einer Konzentration einer flüchtigen Verbindung im Detektor während des Vorgangs der Probenahme,
- Überwachen (M_CVC) eines Maßes, welches die kumulative Summe von flüchtigen organischen Verbindungen als Reaktion auf die erkannte flüchtige organische Verbindung während des Vorgangs der Probenahme misst,
- Vergleichen (C_TH) des besagten Maßes mit einem vorbestimmten Schwellwert, und
- Anhalten (ST_SP) der Probenahme, falls das besagte Maß den vorbestimmten Schwellwert überschreitet.

## Revendications

1. Dispositif d'échantillonnage de gaz agencé pour charger un tube sorbant associé (T) avec des composés organiques volatils provenant d'un échantillon de gaz (G_S) dans un processus d'échantillonnage, le dispositif d'échantillonnage de gaz comprenant
- une entrée de gaz (G_I) agencée pour recevoir l'échantillon de gaz (G_S),
- un port (P) agencé pour recevoir le tube sorbant associé (T),
- un circuit de gaz (GC) connecté à l'entrée de gaz (G_I) et étant agencé pour fournir un chemin d'écoulement pour guider l'échantillon de gaz (G_S) vers le port (P), de façon à permettre à l'échantillon de gaz (G_S) de charger le tube sorbant associé (T),
- au moins un détecteur de composés organiques volatils (DT1) agencé pour détecter le gaz dans le chemin d'écoulement en amont ou en aval du port (P), le détecteur (DT1) étant agencé pour générer un signal de sortie (VC) en fonction d'une concentration de composé organique volatil détectée, et
- un module de commande (CM) connecté à l'au moins un détecteur de composés organiques volatils (DT1), et étant agencé pour calculer une mesure indicative d'une somme cumulée de composés organiques volatils en réponse au signal de sortie (VC) provenant du détecteur (DT1), et pour comparer ladite mesure à une valeur de seuil prédéterminée (TH), et dans lequel le module de commande (CM) est agencé pour générer une sortie (O) dans le cas où ladite mesure dépasse la valeur de seuil prédéterminée (TH).

2. Dispositif d'échantillonnage de gaz selon la revendication 1, comprenant un détecteur de composés organiques volatils (DT1) disposé dans le chemin d'écoulement en amont du port (P).

3. Dispositif d'échantillonnage de gaz selon la revendication 1, comprenant un détecteur de composés organiques volatils (DT2) disposé dans le chemin d'écoulement en aval du port (P).

4. Dispositif d'échantillonnage de gaz selon la revendication 1, comprenant un premier détecteur de composés organiques volatils (DT1) disposé dans le chemin d'écoulement en amont du port (P), et un deuxième détecteur de composés organiques volatils (DT2) disposé dans le chemin d'écoulement en aval du port (P).

5. Dispositif d'échantillonnage de gaz selon la revendication 1, comprenant une pompe contrôlable (PMP) agencée dans le chemin d'écoulement et étant agencée pour générer un écoulement pour forcer l'échantillon de gaz (G_S) de l'entrée de gaz (G_I) au port (P).

6. Dispositif d'échantillonnage de gaz selon la revendication 5, dans lequel la pompe contrôlable (PMP) est agencée pour arrêter de générer ledit flux en réponse à la sortie (O) du module de commande (CM), de manière à arrêter le chargement du tube sorbant associé (T).

7. Dispositif d'échantillonnage de gaz selon la revendication 1, comprenant une valeur contrôlable dans le chemin d'écoulement pour arrêter un écoulement de gaz vers le port (P) en réponse à la sortie (O) du module de commande (CM), de manière à arrêter le chargement du tube sorbant associé (T).

8. Dispositif d'échantillonnage de gaz selon la revendication 1, dans lequel le module de commande (CM) est agencé pour générer une sortie (O) pour indiquer à un utilisateur que le tube sorbant associé (T) est chargé, dans le cas où ladite mesure dépasse la valeur de seuil prédéterminée (TH).

9. Dispositif d'échantillonnage de gaz selon la revendication 1, dans lequel le module de commande (CM) est agencé pour sélectionner entre une pluralité de valeurs de seuil (TH) différentes.

10. Dispositif d'échantillonnage de gaz selon la revendication 9, comprenant un lecteur de code agencé pour lire un code du tube sorbant associé (T), et dans lequel le module de commande (CM) est agencé pour recevoir une sortie du lecteur de code indiquant le code, et pour sélectionner ladite valeur de seuil (TH) en conséquence.

11. Dispositif d'échantillonnage de gaz selon l'une quelconque des revendications 9, comprenant un dispositif d'entrée utilisateur agencé pour recevoir une entrée d'un utilisateur indiquant un type de tube sorbant associé (T), et dans lequel le module de commande (CM) est agencé pour sélectionner ladite valeur de seuil (TH) selon ladite entrée de l'utilisateur.

12. Dispositif d'échantillonnage de gaz selon la revendication 1, comprenant un débitmètre (FM) disposé dans le chemin d'écoulement pour mesurer un volume de gaz passant par le port (P), et pour générer une sortie (V) en conséquence.

13. Dispositif d'échantillonnage selon la revendication 12, dans lequel le module de commande (CM) est agencé pour recevoir la sortie (V) du débitmètre (FM), et pour calculer une valeur indicative d'un volume total (TV) de gaz ayant traversé le port (P) pendant un processus d'échantillonnage, et pour stocker ladite valeur dans une base de données (DB) .

14. Dispositif d'échantillonnage d'haleine portatif comprenant un dispositif d'échantillonnage de gaz selon la revendication 1, dans lequel l'entrée de gaz (G_I) est agencée pour recevoir l'haleine expirée (G_S).

15. Procédé d'échantillonnage de gaz dans un tube sorbant dans un processus d'échantillonnage, le procédé consistant à
- la fourniture d'un dispositif d'échantillonnage de gaz tel que défini dans les revendications 1 à 13,
- recevoir (R_GS) un échantillon de gaz à l'entrée de gaz,
- guider (G_GS) l'échantillon de gaz depuis l'entrée de gaz pour charger le tube sorbant,
- détecter (D_VCC) une concentration de composés organiques volatils avec le détecteur pendant le processus d'échantillonnage,
- surveiller (M_CVC) une mesure indicative d'une somme cumulée de composés organiques volatils en réponse à la concentration de composés organiques volatils détectée pendant le processus d'échantillonnage,
- la comparaison (C_TH) de ladite mesure avec une valeur de seuil prédéterminée, et
- arrêter (ST_SP) le processus d'échantillonnage dans le cas où ladite mesure dépasse la valeur de seuil prédéterminée.
